# EUROPÄISCHE PATENTSCHRIFT

(11) **EP 0 572 592 B1**
(45) Veröffentlichungstag und Bekanntmachung des Hinweises auf die Patenterteilung: **04.06.1997**
(21) Anmeldenummer: 92924533.0
(22) Anmeldetag: 17.12.1992
(51) Int. Cl.: G01N 21/89, G01N 33/36, B65H 63/06

(54) **FREMDFASERERKENNUNG IN GARNEN**
DETECTION OF FOREIGN FIBRES IN YARNS
DETECTION DE FIBRES ETRANGERES DANS DES FILS

(30) Priorität: 20.12.1991 CH 3804/91
(43) Veröffentlichungstag der Anmeldung: 08.12.1993
(73) Patentinhaber: ZELLWEGER LUWA AG, CH-8610 Uster (CH)
(72) Erfinder: GEITER, Paul, CH-8807 Freienbach (CH)
(86) Internationale Anmeldenummer: CH9200245
(87) Internationale Veröffentlichungsnummer: WO9313407

(56) Entgegenhaltungen:
- EP-A- 0 399 945
- WO-A-91/10891
- CH-A- 438 790
- GB-A- 1 211 463
- GB-A- 2 147 096

## Beschreibung

Die Erfindung bezieht sich auf ein Verfahren zur Fremdfasererkennung in Garnen gemäss der Gattung des patentanspruchs 1 sowie auf eine Einrichtung zur Durchführung des Verfahrens gemäss Anspruch 4.

Aus der US-PS 4,739,176 ist bereits ein Verfahren bekannt, bei dem ein bewegtes Garn und ein optisch auf das Garn abgestimmter Hintergrund mit diffusem Licht bestrahlt werden, wobei das Garn und der Hintergrund ungefähr den gleichen Remissionsgrad aufweisen, und das vom Garn und vom Hintergrund remittierte Licht von einem einzigen Sensor detektiert und nach entsprechender Verarbeitung zur Erkennung von Fremdfasern im Garn ausgewertet wird. Bei diesem Verfahren werden für ein Garn ohne Fremdfasern weder Dickstellen noch Dünnstellen gemeldet, da für den Sensor das fremdfaserfreie Garn gegen den Hintergrund verschwindet, er somit keinen Unterschied zwischen dem Garn und dem Hintergrund sieht. Fremdfasern, deren Remissionsgrad bekanntlich stets von dem des Garnes verschieden ist, werden dagegen gemeldet. Dieses bekannte Verfahren ist nur anwendbar, wenn für den Sensor der Remissionsgrad des Hintergrundes möglichst genau mit dem des Garnes übereinstimmt. Geringe Unterschiede in der Farbe bzw. dem Schwarzwert des Garnes führen dazu, dass Dick- oder Dünnstellen als Änderungen des remittierten Lichtes am Sensor festgestellt werden und so Fremdfasern vortäuschen. Die Anwendung dieser Verfahren ist somit im wesentlichen auf die Prüfung ungefärbter Garne, am ehesten bei gleicher Herkunft, beschränkt.

Aus der GB-B 2 095 828 ist ein Verfahren bekannt, bei dem ein bewegtes Faserkollektiv, z.B. in Form eines flächenförmigen Faservlieses, an zwei auseinanderliegenden Stellen mit je einer Lichtquelle beleuchtet und an der einen Stelle das von den Fasern transmittierte, an der anderen Stelle das von den Fasern reflektierte Licht mittels optisch abbildender Systeme je auf einen separaten Lichtsensor geleitet wird. Das von derselben Stelle des Faserkollektives aufgefangene Licht wird von dem in Bewegungsrichtung hinteren Sensor mit einer Zeitverzögerung gegenüber dem vorderen Sensor gemessen, welche vom Abstand der beiden Messstellen und von der Geschwindigkeit des Faserkollektivs abhängt. Auf dieses Weise ist es möglich, Werte sowohl für das Transmissionsvermögen als auch für das Reflektionsvermögen derselben Stelle des Faserkollektivs zu erhalten, welche mit vorgegebenen Sollwerten verglichen werden und eine gewisse Klassifikation der auftretenden Fehler, darunter auch die Feststellung von Fremdkörpern im Faservlies, erlauben. Da über den ganzen Querschnitt des Faservlieses gemessen werden muss, werden als Sensoren Photodetektoren in der Form von teuren Zeilenarrays verwendet, was auch eine aufwendige Auswerteelektronik bedingt. Deshalb ist diese Einrichtung nur zur Prüfung von Materialproben, nicht aber in der Fertigung verwendbar. Bei diesem bekannten Verfahren können gewisse Fehlinterpretationen der Messwerte dadurch entstehen, dass die Messwerte, aus denen auf die Anwesenheit einer Fremdfaser geschlossen werden, an verschiedenen Stellen und damit zeitlich nacheinander gewonnen werden. Dies gilt im besonderen beim Anfahren und beim Herunterfahren der überwachten Fertigungsstelle, da sich dabei die Geschwindigkeit des Garnes innerhalb der Zeitverzögerung zwischen den zusammengehörigen Messwerten ändert.

Gegen die Fehlermöglichkeiten beider bekannter Verfahren will die Erfindung Abhilfe schaffen. Der Erfindung liegt die Aufgabe zugrunde, ein Verfahren zum Erkennen von Fremdfasern in Garnen zu schaffen, das unabhängig von der Farbe des Garnes ist und bei dem Messwerte, aus denen auf die Anwesenheit einer Fremdfaser an einer Stelle des bewegten Garnes geschlossen werden kann, gleichzeitig und an derselben Stelle messbar sind.

Diese Aufgabe wird erfindungsgemäss durch die Merkmale des Anspruchs 1 gelöst. Weitere vorteilhafte Ausgestaltungen der Erfindung sind in den abhängigen Ansprüchen gekennzeichnet.

Ein Ausführungsbeispiel der Erfindung, welches zugleich das Funktionsprinzip erläutert, ist in der Zeichnung dargestellt und wird im folgenden näher beschrieben. Es zeigen:
- Fig. 1: eine perspektivische Zeichnung einer erfindungsgemässen Einrichtung zur Fremdfasererkennung;
- Fig. 2: einen Schnitt parallel zur Draufsicht der Fig. 1, entsprechend dem Schnitt B - B der Fig. 3;
- Fig. 3: einen Schnitt A - A der Fig. 2;
- Fig. 4: einen Schnitt C - C der Fig. 3;
- Fig. 5: ein Blockschaltbild einer erfindungsgemässen Einrichtung;
- Fig. 6: Signalverläufe bei der erfindungsgemässen Einrichtung;
- Fig. 7: einen Schnitt längs der Linie B - B der Fig. 8 durch eine modifizierte erfindungsgemässen Einrichtung zur Fremdfasererkennung;
- Fig. 8: einen Schnitt längs der Linie A - A der Fig. 7 durch eine modifizierte erfindungsgemässen Einrichtung zur Fremdfasererkennung; und
- Fig. 9: einen Schnitt längs der Linie C - C der Fig. 8 durch eine modifizierte erfindungsgemässen Einrichtung zur Fremdfasererkennung.

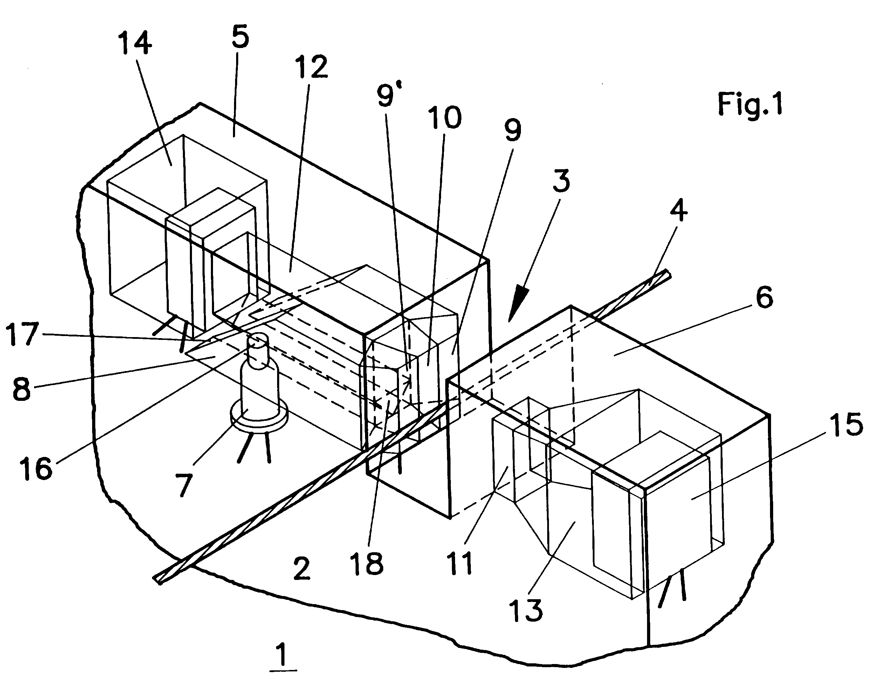

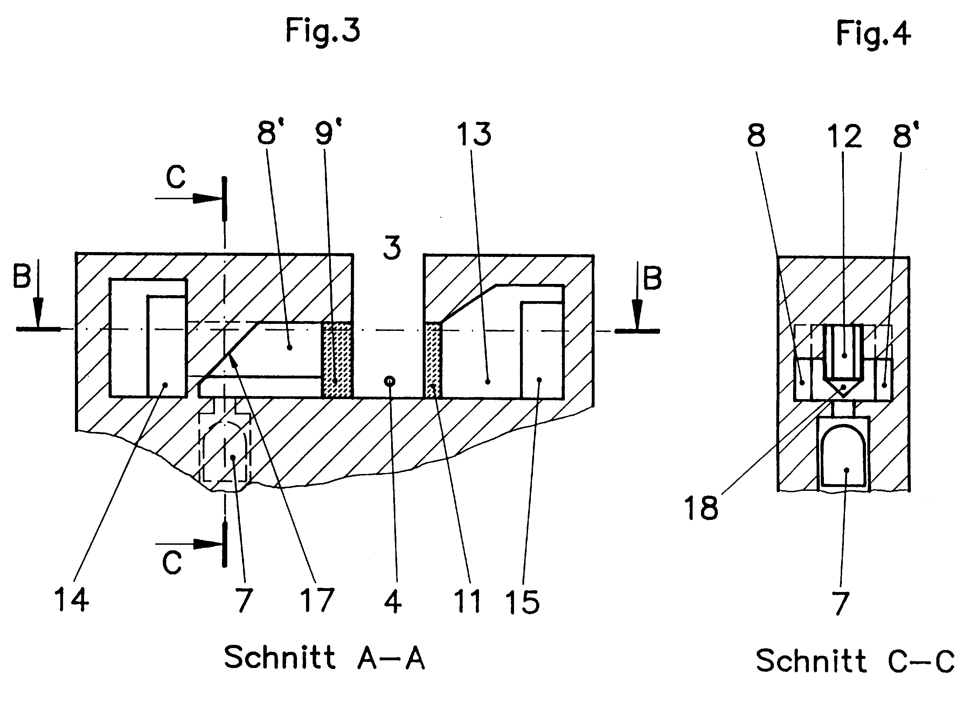

In allen Figuren haben entsprechende Teile dieselben Beizeichen. Die in den Figuren 1 bis 4 dargestellte Einrichtung zur Fremdfasererkennung 1 (im folgenden kurz "Einrichtung" genannt), sitzt in einem Gehäuse 2, das einen Kanal 3 zum Durchlauf eines Garnes 4 oder eines anderen fadenförmigen Gebildes besitzt und für Baugruppen dient, wie sie an Spinnstationen oder dergleichen verwendet werden. Das Garn 4 ist durch nicht gezeichnete Führungseinrichtungen so geleitet, dass es quer zu seiner Laufrichtung nicht wesentlich ausweichen kann.

In einem Quellenteil 5 des Gehäuses 2 befindet sich eine Lichtquelle 7, beispielsweise eine Leuchtdiode, ein erster Lichtzuführer 8, der Licht von der Lichtquelle 7 durch später zu beschreibende Mittel in zwei Bündel aufteilt und es über zwei Eintrittsprismen 9, 9' auf das Garn 4 wirft. Das vom Garn 4 reflektierte Licht wird über ein erstes Austrittsprisma 10 und einen zweiten Lichtzuführer 12 einem ersten Sensor 14 für Licht zugeführt. Die Ausdrücke "Eintritt" und "Austritt" sind auf das Garn 4 bezogen.

In einem Sensorteil 6 des Gehäuses befindet sich ein zweites Austrittsprisma 11 und ein dritter Lichtzuführer 13, die das durch das Garn 4 transmittierte Licht einem zweiten Sensor 15 für Licht zuführen. Der Kanal 3 zum Durchlauf des Garnes 4 liegt zwischen dem Quellenteil 5 und dem Sensorteil 6 des Gehäuses 2.

Die Lichtzuführer 8, 12, 13 sind vorzugsweise als innen reflektierende, beispielsweise verspiegelte Hohlräume ausgebildet, in denen sich das Licht fortpflanzt. Der erste Lichtzuführer 8 besitzt ein Lichteintrittsfenster 16 über der Lichtquelle 7, eine reflektierende Schräge 17, die das aus der Lichtquelle 7 nach oben austretende Licht in Garnrichtung wirft, und einen Strahlenteiler 18, der dann das Licht auf die Eintrittsprismen 9, 9' lenkt. Von den Eintrittsprismen 9,9' wird das Licht so abgelenkt, dass es sich auf einem Fleck am Garn 4 konzentriert, der einen Durchmesser wenig grösser als die durch die Führungseinrichtungen beschränkten Auslenkungen des Garnes 4 besitzt.

Die Austrittsprismen 10, 11 sammeln jeweils das reflektierte bzw. das transmittierte Licht aus diesem Fleck und leiten es über die zugehörigen Lichtzuführer 12, 13 den Sensoren 14, 15 zu.

Die Lichtzuführer 8, 12, 13, die zugehörigen Eintrittsprismen 9, 9' bzw. die Austrittsprismen 10, 11 werden, wo notwendig, unter dem Begriff "lichtzuführende Mittel" zusammengefasst. Diese können aber auch als Lichtleiter oder in irgend einer anderen Form, in der sie das Licht fortpflanzen, ausgebildet sein.

Die Einrichtung 1 zur Fremdfasererkennung kann vor einem Garnreiniger angebracht werden. Mit dieser Kombination ist es dann möglich, alle Stücke aus dem Garn 4 herauszuschneiden, die Fremdfasern enthalten und das Garn dann neu zu spleissen.

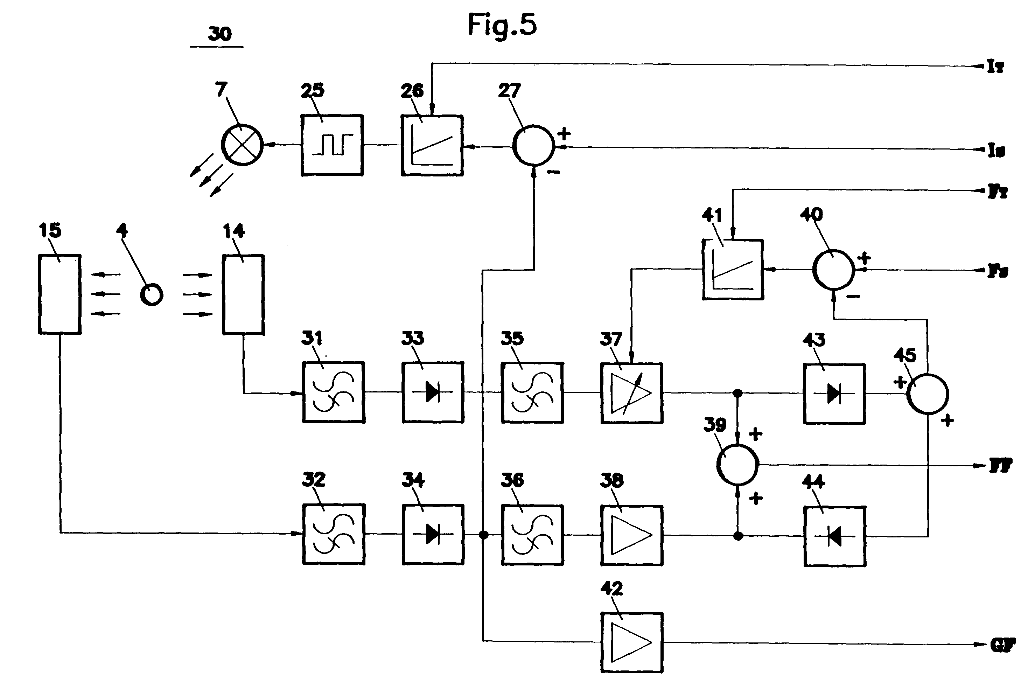

Ein Beispiel für die elektronischen Mittel 30 der Einrichtung 1 gibt die Fig. 5 wieder. Es zeigt die Lichtquelle 7, die Licht auf das Garn 4 wirft, und den ersten Sensor 14 für das vom Garn 4 reflektierte Licht sowie den zweiten Sensor 15 für das Licht, welches das Garn transmittiert.

In die Versorgung der Lichtquelle 7 ist ein Modulator 25 geschaltet, der das Licht mit einer gegebenen Frequenz ein- und ausschaltet, so dass an den Sensoren 14, 15 nur der dadurch entstehende Wechselanteil für die Weiterverarbeitung der Signale massgeblich ist. Vor dem Modulator 25 ist ein erster Regler 26 geschaltet, mit dem, wie später gezeigt wird, die Leistung der Lichtquelle 7 eventuellen Verschmutzungen der Messstrecke angepasst werden kann.

Vom ersten Sensor 14 geht ein elektrisches Signal für die Intensität des reflektierten Lichtes über ein erstes Hochpassfilter 31, einen ersten Demodulator 33, ein zweites Hochpassfilter 35 und einen variablen Verstärker 37 auf einen Summierer 39. Vom zweiten Sensor 15 geht ein elektrisches Signal für die Intensität des transmittierten Lichtes über ein drittes Hochpassfilter 32, einen zweiten Demodulator 34, ein viertes Hochpassfilter 36 und einen konstanten Verstärker 38 auf denselben Summierer 39.

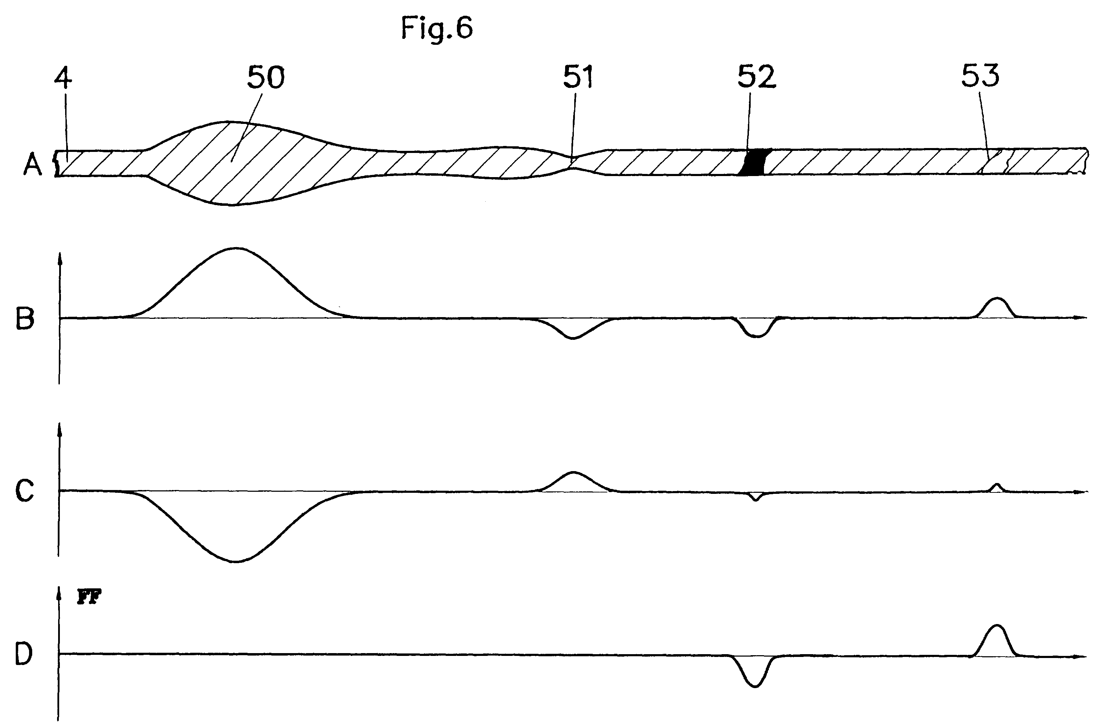

Das erste und der dritte Hochpassfilter 31 bzw. 32 eliminiert jeweils den Anteil des die Sensoren 14 bzw. 15 treffenden Umgebungslichtes auf das Signal für das reflektierte bzw. das transmittierte Licht und lässt nur den modulierten Anteil des Nutzsignals durch. Die Demodulatoren 33 bzw. 34 richten diese modulierten Anteile gleich. Es entstehen Gleichstromsignale, die nur langsamen Änderungen des die Sensoren 14, 15 treffenden Lichtes folgen, aber von schnellen Änderungen überlagert werden, die durch Änderungen des Reflektionsvermögens bzw. des Transmissionsvermögens durch Fehler am Garn 4 erzeugt werden. Das zweite und vierte Hochpassfilter 35, 36 eliminiert den Gleichstromanteil dieser Signale und lässt nur die schnellen Änderungen durch. Diese werden über die Verstärker 37 bzw. 38 auf den Summierer 39 gegeben. Damit wird im Summierer 39 jeweils die Summe der Änderungen des reflektierten und des transmittierten Lichtes gebildet. Diese Grösse wird über den Ausgang FF ausgegeben und meldet, wie wir bei der Besprechung der Fig. 6 sehen werden, jeweils die Anwesenheit einer Fremdfaser.

Für die Regelung des Verstärkers 37 sollten die Ausgangssignale der Verstärker 37 und 38 gleichgerichtet werden. Der Gleichrichter 43 liefert ein negatives, und der Gleichrichter 44 ein positives Ausgangsignal. Diese beiden gleichgerichteten Signale werden im Summierer 45 summiert. Der Ausgang des Summierers 45 geht an den negativen Eingang eines zweiten Vergleichers 40, an dessen positivem Eingang ein Sollwert FS liegt, der dem Wert "keine Fremdfaser vorhanden" entspricht. Der Ausgang des zweiten Vergleichers 40 ist als Regeleingang an einen zweiten Regler 41 geschaltet, dessen Regelausgang am Steuereingang des variablen Verstärker 37 liegt, der das vom reflektierten Licht abgeleitete Signal endverstärkt. Dadurch gleicht der Verstärker 37 Signale über eine Reflexionsänderung derart denjenigen über eine Transmissionsänderung an, dass die Summe der Signale über die Reflexion und die Transmission Null wird, so lange keine Fremdfaser gemeldet ist. Dabei ist die Zeitkonstante des zweiten Reglers 41 so zu wählen, dass langfristige Änderungen am Garn 4 in der Verstärkung ausgeregelt werden, kurzfristige Änderungen aber wirksam werden.

Das Signal nach dem zweiten Demodulator 34, das als Gleichstrom dem von der Umgebungsstrahlung befreiten transmittierten Licht entspricht, wird an den negativen Ausgang eines ersten Vergleichers 27 gegeben. Am positiven Ausgang des ersten Vergleichers 27 liegt ein Sollwert IS für die Intensität der Lichtquelle 7. Der Ausgang des ersten Vergleichers 27 wird auf den Eingang eines ersten Reglers 26 gegeben, der mit einer so grossen Zeitkonstante arbeitet, dass durch transmittiertes Licht gemeldete Garnfehler den ersten Regler 26 nicht beeinflussen. Die den ersten Regler 26 beeinflussenden Änderungen im transmittierten Licht können daher nur von Verschmutzungen im Strahlengang herrühren, die Intensität der Lichtquelle 7 wird durch den ersten Regler 26 unter Berücksichtigung der Verschmutzung konstant gehalten.

In Fig. 5 ist ein zusätzliches Ausgangssignal GF mit dem Verstärker .42 dargestellt. Der Ausgang GF liefert ein Garnsignal, wie es vom herkömmlichen Garnreiniger her bekannt ist. Damit besteht die Möglichkeit, den Fremdfaser-Tastkopf grundsätzlich mit dem Reinigertastkopf zu kombinieren.

Die Art der Signalbildung wird in der Fig. 6 gezeigt. Hier ist unter A ein Garn 4 gezeigt, das nacheinander eine Dickstelle 50, eine Dünnstelle 51, eine dunkle Fremdfaser 52 und eine helle Fremdfaser 53 aufweist.

Unter B wird das Signal gezeigt, das am Ausgang des variablen Verstärkers 37 gebildet wird. Es stammt von dem Sensor 14 für das reflektierte Licht. Der Umgebungslichteinfluss ist eliminiert, Im Demodulator 33 ist es in ein Gleichstromsignal gewandelt worden und dann durch das zweite Hochpassfilter 35 und den variablen Verstärker 37 gelaufen, so dass es nur noch die kurzzeitigen Änderungen des reflektierten Lichtes anzeigt. An der Dickstelle 50 zeigt es einen grossen positiven Ausschlag, da dort die reflektierende Fadenfläche gross ist. Der Ausschlag an der Dünnstelle 51 ist klein und negativ, da dort die reflektierende Fadenfläche verkleinert ist. Die dunkle Fremdfaser 52 zeigt sich als deutlicher, negativer Ausschlag, da die Fremdfaser 52 dort gegenüber dem normalen Zustand mehr Licht absorbiert und somit der Reflexionsgrad der Faser herabgesetzt ist. Die helle Fremdfaser 53 absorbiert weniger Licht, der Ausschlag ist deutlich positiv.

Unter C wird das Signal gezeigt, wie es am Ausgang des konstanten Verstärkers 38 gebildet wird. Es stammt von dem zweiten Sensor 15 für transmittiertes Licht und hat dieselben Funktionen durchlaufen wie oben aufgeführt. An der Dickstelle 50 zeigt es einen grossen negativen Ausschlag, da dort eine grössere Fadenfläche für das transmittierte Licht abdeckend wirkt. Der Ausschlag an der Dünnstelle 51 ist klein, aber positiv, da dort die Abdeckung geringer wird. Die dunkle Fremdfaser 52 absorbiert auch transmittiertes Licht, man erhält, wie unter B, einen deutlichen, negativen Ausschlag. Die helle Fremdfaser 53 absorbiert weniger Licht als das übrige Garn 4, der Ausschlag ist deutlich positiv.

Unter D wird das Signal FF am Ausgang des Summierers 39 gezeigt. Dieser unterdrückt an derselben Stelle des Garnes 4 auftretende Ausschläge von B und C mit unterschiedlichen Vorzeichen und hebt Ausschläge hervor, die das gleiche Vorzeichen haben. Deswegen werden die Dickstelle 50 und die Dünnstelle 51 unterdrückt, die helle Fremdfaser 53 und die dunkle Fremdfaser 52 werden angezeigt. Die erfindungsgemässe Einrichtung zeigt somit Fremdfasern 52,53 an, unterdrückt aber die sonstigen Garnfehler. Dabei ist die Einrichtung wegen der Wirkung des variablen Verstärkers 37 selbstverständlich auch unabhängig von der Grundfärbung des Garnes.

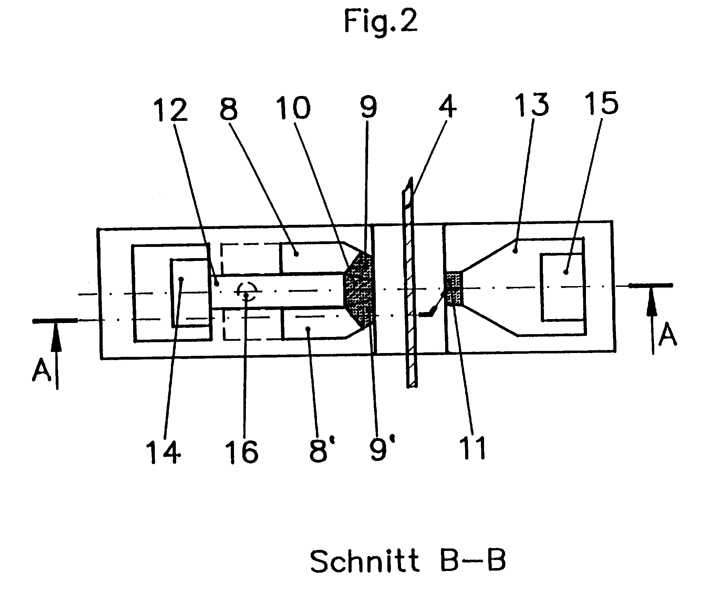

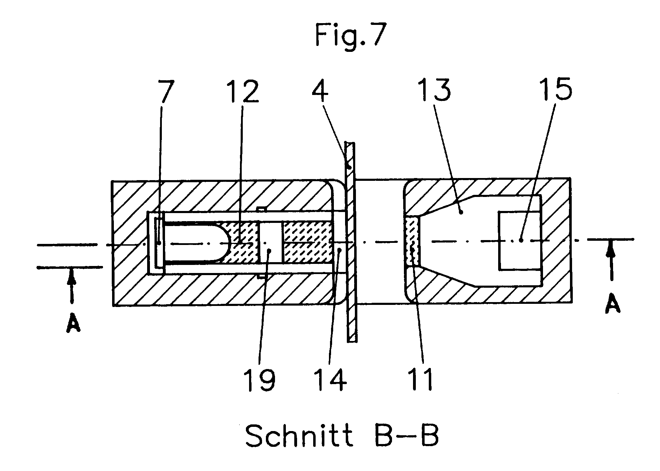

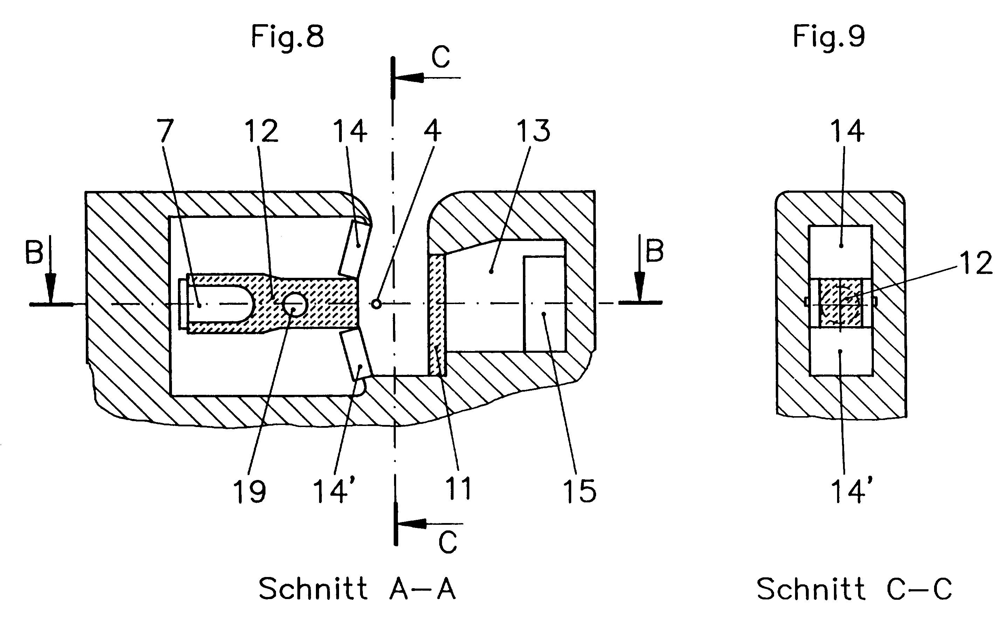

In den Fig. 7 - 9 (welche den Fig. 2 - 4 der oben beschriebenen Grundausführung der Erfindung entsprechen) ist eine solche Modifikation der erfindungsgemässen Vorrichtung dargestellt, bei welcher die Sende/Empfangskonfiguration aus einer Lichtquelle 7, zwei Lichtsensoren 14,14' für reflektiertes Licht und einem Lichtsensor 15 für transmittiertes Licht besteht. Bei dieser Ausführungsform ist im Lichtzuführer 12 ein Loch 19 vorgesehen, welches wegen seiner groben Oberfläche als Diffusor wirkt. Damit wird das Licht der Lichtquelle 7 im Messfeld besser verteilt. Die beiden Lichtsensoren 14,14' für reflektiertes Licht ermöglichen eine bessere Messung des reflektierten Lichtes, d.h. das Nutzsignal ist grösser und die kontrollierte Oberfläche des Garnes ist ebenfalls grösser.

Das erfindungsgemässe Verfahren kann durch den Einsatz von Licht unterschiedlicher Wellenlänge weiter verbessert werden. Fremdfasern können sich durch die Farbe, durch die chemische Zusammensetzung oder durch beide dieser Merkmale von den gewünschten Fasern unterscheiden. Die Farbabweichung kann mit sichtbarem Licht erfasst werden. Um ein universelles Verfahren zur Erkennung von Fremdfasern zu erhalten, welches nicht nur bei einer bestimmten Garnfarbe funktioniert, muss eine minimale spektrale Messung durchgeführt werden. Dazu sind zwei Wege möglich:
1. Die spektrale Messung erfolgt mit Lichtsensoren 14,15 die nur für einen bestimmten Wellenlängenbereich des Lichts empfindlich sind. Dabei kann für den Sensor 15 für das transmittierte Licht eine andere Wellenlänge als für den Sensor 14 für das reflektierte Licht gewählt werden. Die Lichtquelle 7 dagegen emittiert Licht, welches alle Wellenlängenbereiche der verwendeten Lichtsensoren 14,15 abdeckt.
2. Die spektrale Messung kann aber mittels mehrerer Lichtquellen 7 mit engem Wellenlängenbereich durchgeführt werden, wobei diese nacheinander Licht in verschiedenen Wellenlängebereichen emittieren und die Lichtsensoren 14,15 auf sämtliche emittierten Wellenlängen empfindlich sind. Vorzugsweise werden zwei Lichtquellen 7 eingesetzt, deren Wellenlängenbereiche sich nicht überschneiden. Bei nur einer Lichtquelle 7 können Farbveränderungen innerhalb des emittierten Wellenlängebereichs nicht erfasst werden. Auch Fremdfasern deren Farbe mit dem emittierten Licht übereinstimmen können in einem weissen Garn nicht detektiert werden. Dieser sogenannte "blinde" Bereich kann durch den Einsatz einer anderen Lichtquelle 7 abgedeckt werden. Zwei Lichtquellen 7 mit unterschiedlichen Wellenlängenbereichen können somit Farbveränderungen innerhalb des gesamten sichtbaren Spektrums erfassen.

Wenn bei der gleichen Konfiguration eine Lichtquelle 7 im nahen Infrarotbereich (NIR) eingesetzt wird, dann können Abweichungen in der chemischen Zusammensetzung erfasst werden. Baumwolle, die vorwiegend aus Zellulose besteht, zeigt eine typische spektrale Reflexionslinie bei ca. 1600 nm, die bei synthetischen Fasern, Wollfasern oder anderen Fremdkörpern fehlt. Das Signal des Lichtsensors wird abnehmen, wenn anstelle der Baumwollfaser eine chemisch anders zusammengesetzte Fremdfaser oder Fremdpartikel erscheint.

Damit ist die Aufgabe gelöst, eine Einrichtung zu schaffen, mit der durch ortsfeste Messung an einer Stelle eines bewegten Garnes Fremdfasern im Garn unabhängig von der Garnfärbung feststellbar sind.

## Patentansprüche

1. Verfahren zur Erkennung von Fremdfasem (52, 53) in längsbewegten textilen Gamen (4) bei welchem Licht von einer oder mehreren Lichtquellen (7) auf das Gam geworfen wird, das vom Gam (4) reflektierte Licht mindestens einem ersten Sensor (14), das im Gam (4) transmittierte Licht mindestens einem zweiten Sensor (15) zur Bildung von Messwerten für die Intensität des Lichts zugeführt wird und aus diesen Messwerten mit Hilfe elektronischer Mittel (30) das Vorhandensein von Fremdfasem (52, 53) im Gam (4) feststellbar ist, dadurch gekennzeichnet, dass das Licht der Lichtquellen (7) an einer einzigen ortsfesten Stelle auf das bewegte Gam (4) geworfen wird, wo es gleichzeitig reflektiert und transmittiert wird, dass gleichzeitig das reflektierte Licht vom ersten Sensor (14) und das transmittierte Licht vom zweiten Sensor (15) jeweils in ein elektrisches Signal gewandelt wird und dass aus den elektrischen Signalen vom ersten und vom zweiten Sensor durch Summierung ein weiteres elektrisches Signal (FF) gewonnen wird, bei dem die sonstigen Gamfehler unterdrückt und die Fremdfasem angezeigt werden.

2. Verfahren nach Anspruch 1, dadurch gekennzeichnet, dass das auf das Gam (4) geworfene Licht moduliert ist und die von den Sensoren (14, 15) ausgehenden elektrischen Signale sowohl für reflektiertes wie auch für transmittiertes Licht durch die elektronischen Mittel (3) so gewandelt werden, dass sie jeweils nur die Abweichungen von einem an fehlerfreiem Gam (4) gemessenen Wert darstellen und diese Abweichungen einem Summierer (39) zugeführt werden, dessen Ausgang die Anwesenheit einer Fremdfaser im Gam anzeigt.

3. Verfahren nach Anspruch 2, dadurch gekennzeichnet, dass die Signale für das reflektierte Licht vom ersten Sensor (14) bzw. für das transmittierte Licht vom zweiten Sensor (15) nacheinander durch ein erstes bzw. ein drittes Hochpassfilter (31 bzw. 32), die jeweils den Anteil des die Sensoren (14 bzw. 15) treffenden Umgebungslichtes eliminieren, durch Demodulatoren (33 bzw. 34), die diese modulierten Anteile gleichrichten, durch ein zweites bzw. ein viertes Hochpassfilter (35 bzw. 36), die den Gleichstromanteil dieser Signale eliminieren und nur die schnellen Änderungen durchlassen und durch Verstärker (37 bzw. 38) hindurchgehen und auf den Summierer (39) gegeben werden, wo Signale über Anwesenheit von Fremdfasem gebildet werden.

4. Einrichtung zur Erkennung von Fremdfasem (52, 53) in längsbewegten textilen Garnen (4), bei welcher Licht von einer oder mehreren Lichtquellen (7) auf das Gam geworfen wird, das vom Garn (4) reflektierte Licht mindestens einem ersten Lichtsensor (14), das im Gam (4) transmittierte Licht mindestens einem zweiten, Lichtsensor (15) zur Bildung von Messwerten für die Intensität des Lichts zugeführt wird, und aus diesen Messwerten mit Hilfe elektronischer Mittel (30) das Vorhandensein von Fremdfasem (52, 53) im Gam (4) feststellbar ist, dadurch gekennzeichnet, dass das Licht der Lichtquellen (7) an einer einzigen ortsfesten Stelle auf das bewegte Garn (4) geworfen wird, wo es gleichzeitig reflektiert und transmittiert wird, dass gleichzeitig das reflektierte Licht vom ersten Sensor (14) und das transmittierte Licht vom zweiten Sensor (15) jeweils in ein elektrisches Signal gewandelt wird und dass der erste Lichtsensor und der zweite Lichtsensor an einen Summierer (39) angeschlossen sind.

5. Einrichtung nach Anspruch 4, dadurch gekennzeichnet, dass lichtzuführende Mittel (8, 12, 13; 9, 9', 10, 11) zwischen der Lichtquelle (7) und einer ortsfesten Stelle am bewegten Gam (4) und zwischen dieser Stelle und den Sensoren (14, 15) vorgesehen sind.

6. Einrichtung nach Anspruch 5, dadurch gekennzeichnet, dass die lichtzuführenden Mittel aus Lichtzuführem (8, 12, 13), die als innen reflektierende Hohlräume ausgebildet sind, und Eintrittsprismen (9, 9') bzw. Austrittsprismen (10, 11) bestehen.

7. Einrichtung nach Anspruch 6, dadurch gekennzeichnet, dass ein erster Lichtzuführer (8), der das Licht von der Lichtquelle (7) über die Einrittsprismen (9, 9') zum Gam (4) leitet, ein Lichteintrittsfenster (16), eine reflektierende Schräge (17) und einen Strahlenteiler (18) besitzt.

8. Einrichtung nach Anspruch 5, dadurch gekennzeichnet, dass die lichtzuführenden Mittel Lichtleiter beliebiger Art sind.

9. Einrichtung nach Anspruch 4, dadurch gekennzeichnet, dass der Lichtquelle ein Modulator (25) zu dessen Speisung vorgeschaltet ist.

10. Einrichtung nach Anspruch 9, dadurch gekennzeichnet, dass der Modulator (25) mit einem Regler (26), dessen Zeitkonstante veränderbar ist, und mit einem Vergleicher (27) verbunden ist, der einen Sollwert IS der Lichtintensität mit dem Ausgangswert des Demodulators (34) vergleicht.

11. Einrichtung nach Anspruch 4, dadurch gekennzeichnet, dass der erste Sensor (14) und der zweite Sensor (15) über ein erstes bzw. drittes Hochpassfilter (31, 32) und einen ersten bzw. zweiten Demodulator (33 bzw. 34) mit einem zweiten bzw. einem vierten Hochpassfilter (35 bzw. 36) verbunden sind und dass diese über einen Verstärker (37 bzw. 38) mit Eingängen eines Summierers (39) verbunden sind.

12. Einrichtung nach Anspruch 11, dadurch gekennzeichnet, dass der Ausgang FF des Summierers (39) über einen Vergleicher (40), der den Wert eines Signals "keine Fremdfaser anwesend" FS mit dem Ausgang des Summierers (39) vergleicht, an einen zweiten Regler (41) angeschlossen ist, der den variablen Verstärker (37) regelt.

13. Einrichtung nach Anspruch 4, dadurch gekennzeichnet, dass in Bewegungsrichtung des Games (4) nachfolgend ein Gamreiniger vorgesehen ist, der zum Ausschneiden von Gamstücken mit Fremdfasem und zum Spleissen von geschnittenen Gamstücken mit den elektronischen Mitteln (30) verbunden ist.

14. Einrichtung nach Anspruch 4, dadurch gekennzeichnet, dass eine einzige Lichtquelle (7) vorgesehen ist, vorzugsweise mit einem breitbandigen Emissionsspektrum.

15. Einrichtung nach Anspruch 14, dadurch gekennzeichnet, dass mehrere, vorzugsweise parallel arbeitende Lichtsensoren (14) für reflektiertes Licht vorgesehen sind.

16. Einrichtung nach Anspruch 15, dadurch gekennzeichnet, dass die Lichtsensoren (14) in voneinander verschiedenen Wellenlängenbereichen empfindlich sind.

17. Einrichtung nach Anspruch 16, dadurch gekennzeichnet, dass mehrere Lichtquellen (7), vorzugsweise zwei Lichtquellen (7) vorgesehen sind.

18. Einrichtung nach Anspruch 17, dadurch gekennzeichnet, dass jede Lichtquelle (7), vorzugsweise zeitlich gestaffelt, in einem eigenen Wellenlängenbereich emittiert.

19. Einrichtung nach Anspruch 18, dadurch gekennzeichnet, dass ein einziger, entsprechend breitbandiger Lichtsensor (14) für reflektiertes Licht vorgesehen ist.

20. Einrichtung nach Anspruch 18, dadurch gekennzeichnet, dass zu jeder in einem eigenen Wellenlängenberich emittierenden Lichtquelle (7) ein korrespondierender Lichtsensor (14) für reflektiertes Licht vorgesehen ist.

21. Einrichtung nach einem der Ansprüche 14 - 20, dadurch gekennzeichnet, dass ein Lichtsensor (15) für transmittiertes Licht vorgesehen ist, der in einem anderen Wellenlängenbereich empfindlich ist als die Lichtsensoren (14) für reflektiertes Licht.

## Claims

1. Method for detecting foreign fibres (52, 53) in lengthwise moving textile yams (4) whereas the yam is exposed to light directed from one or several light sources (7), light reflected by the yam is directed to at least one first sensor (14) and light transmitted by the yam is directed to at least one second sensor (15) for generating measured values representing the intensity of the light and whereas these values are used in electronic means (30) for determining the presence of foreign fibres (52, 53) in the yam (4), characterised in that the light emitted by the light sources is directed to one fixed position only on the moving yam (4) where light is reflected and transmitted at the same time, in that reflected light is converted in the first sensor (14) and transmitted light is converted in the second sensor (15) into an electrical signal and in that a further electrical signal (FF) is obtained by adding the electrical signals from the first and second sensors, wherein usual yam imperfections are suppressed and foreign fibres are shown.

2. Method according to claim 1, characterized in that the light directed to the yam (4) is modulated and the signals produced by the sensors (14, 15) for reflected and transmitted light are converted by the electronic means (30) to only show deviations from values measured on a fault free yam (4) and in that said deviations are fed to an adder (39) having an output showing the presence of a foreign fibre in the yam.

3. Method according to claim 2, characterized in that the signals from the first sensor (14) representing reflected light and the second sensor (15) representing transmitted light are first treated in a first or third high-pass filter (31 resp. 32) thereby liminating such part of the signal corresponding to ambient light received by the sensors (14, 15), then in a demodulator (33 resp. 34) which rectifies these modulated parts, then in a second or fourth high-pass filter (35 resp. 36) which eliminates a direct current component and only transmits fast deviations, and finally in an amplifier (37 resp. 38) and then are fed to the adder (39) where signals indicating the presence of foreign fibres are issued.

4. Apparatus for detecting foreign fibres (52, 53) in lengthwise moving textile yams (4) wherein the yam is exposed to light directed from one or several light sources (7), light reflected by the yam is directed to at least one first sensor (14) and light transmitted by the yam is directed to at least one second sensor (15) for generating measured values representing the intensity of the light and wherein these values are used in electronic means (30) for determining the presence of foreign fibres (52, 53) in the yam (4), characterised in that the light emitted by the light sources is directed to one fixed position only on the moving yam (4) where light is reflected and transmitted at the same time, in that reflected light in the first sensor (14) and transmitted light in the second sensor (15) is each converted into an electrical signal and in that the first sensor and the second are both connected to an adder (39).

5. Apparatus according to claim 4, characterised in that light transmitting means (8, 12, 13, 9, 9', 10, 11) are provided between the light source (7) and one fixed position anlong the moving yam (4) and between this position and the sensors (14, 15).

6. Apparatus according to claim 5, characterised in that said light transmitting means are constituted by light transmitters (8, 12, 13) having inside reflecive cavities and inlet prismes (9, 9') or outlet prismes (10, 11).

7. Apparatus according to claim 6, characterised in that a first light transmitter (8) which guides the light from the source (7) through the inlet prismes (9, 9') to the yam (4) comprises an entry window (16), an inclined reflective face (17) and a beam splitter (18).

8. Apparatus according to claim 5, characterised in that the light transmitting means are photoconductors of any kind.

9. Apparatus according to claim 4, characterised in that the light source (7) is series connected to a modulator (25) for power supply.

10. Apparatus according to claim 9, characterised in that the modulator (25) is connected to a controller (26) whose time constant is adjustable and to a comparator (27) which compares a set value IS of the intensity of light to an output value of the demodulator (34).

11. Apparatus according to claim 4, characterised in that the first sensor (14) and the second sensor (15) are connected through a first or third high-pass filter (31, 32) and a first or second demodulator (33 or 43) to a second or fourth high-pass filter (35, 36) and that these elements are connected through an amplifier (37 or 38) to inputs of an adder (39).

12. Apparatus according to claim 11, characterised in that the output (FF) of the adder (39) is connected through a comparator (40), which compares the value of a signal FS indicating that no foreign fibre is present with the value at the output of the ad- der (39), to a second controller (41) which controlls the adjustable amplifier (37).

13. Apparatus according to claim 4, characterised in that a yam cleaner is provided downstreams of the moving yam (4), which is connected to electronic means (30) for cutting out yam portions containing foreign fibres and for splicing of separated yam portions.

14. Apparatus according to claim 4, characterised in that there is provided a single light source (7), preferably having a wide-band emission spectrum.

15. Apparatus according to claim 14, characterised in that there is provided a plurality of sensors (14) for reflected light preferably for parallel operation.

16. Apparatus according to claim 15, characterised in that the sensors (14) have sensitivities related to different ranges of wavelengths.

17. Apparatus according to claim 16, characterised in that there are provided several light sources (7), preferably two of them.

18. Apparatus according to claim 17, characterised in that each light source (7) emits light in its own range of wave lengths and preferably on different times.

19. Apparatus according to claim 18, characterised in that there is provided one single wide-band light sensor (14) for reflected light.

20. Apparatus according to claim 18, characterised in that for each light source (7) emitting in its own range of wave lengths there is provided a corresponding light sensor (14) for reflected light.

21. Apparatus according to one of the claims 14 - 20, characterised in that there is provided a light sensor (15) for transmitted light which is sensitive in another range of wave lengths than the sensors (14) for reflected light.

## Revendications

1. Procédé de détection de fibres étrangères (52, 53) dans des fils textiles (4) en mouvement en sense longitudinal où de la lumière est dirigée vers le fil à partir d'une ou plusieurs sources (7), où la lumière réfléchie par le fil (4) est dirigée vers au moins un premier capteur (14), où la lumière transmise par le fil (4) est dirigée vers au moins un deuxième capteur (15) afin de produire des valeurs de mesure qui représentent l'intensité de la lumière et où la présence de fibres étrangères (52, 53) dans le fil (4) est signalée par des moyens (30) électroniques, caractérisé en ce que la lumière venant des sources (7) est dirigée sur le fil (4) en mouvement en un seul endroit fixe d'où elle est à la fois réfléchie et transmise et en ce que la lumière réfléchie est convertie en un signal électrique dans le premier capteur (14) et la lumière transmise est convertie en un signal électrique dans le second capteur (15) et en ce que
un autre signal électrique (FF) qui indique les fibres étrangères mais dissimule les autres irrégularités du fil est crée en additionnant les signaux électriques venant du premier et du second capteur.

2. Procédé selon la revendication 1, caractérisé en ce que la lumière dirigée vers le fil (4) est modulée et que les signaux électriques partant des capteurs (14, 15) sont convertis par les moyens électroniques (30) aussi bien pour la lumière réfléchie que la lumière transmise afin d'indiquer uniquement les déviations par rapport à une valeur mesuré sur un fil parfait et en ce que ces déviations sont amenées à un additionneur (39) dont la sortie indique la présence d'une fibre étrangère.

3. Procédé selon la revendication 2, caractérisé en ce que les signaux de la lumière réfléchie venant du premier capteur (14) et de la lumière transmise venant du second capteur (15) sont traités dans un premier ou troisième filtre passe-haut (31 resp. 32) qui élimine la part des signaux due à la lumière ambiante captée par les capteurs (14, 15), dans un démodulateur (33 resp. 34) qui redresse ces parts modulées, dans un deuxième ou quatrième filtre passe-haut (35 ou 36) qui élimine la part de courant continu dans ces signaux mais ne laisse passer que les variations rapides et dans un amplificateur (37 ou 38) et sont ensuite amenés à l'additionneur (39) où des signaux qui indiquent la présence de fibres étrangères sont produits.

4. Appareil de détection de fibres étrangères (52, 53) dans des fils textiles (4) en mouvement en sense longitudinal où de la lumière est dirigée vers le fil à partir d'une ou plusieures sources (7), où
la lumière réfléchie par le fil (4) est dirigée vers au moins un premier capteur (14), où la lumière transmise par le fil (4) est dirigée vers au moins un deuxième capteur (15) afin de produire des valeurs de mesure qui représentent l'intensité de la lumière et où la présence de fibres étrangères (52, 53) dans le fil (4) est signalée par des moyens (30) électroniques, caractérisé en ce que la lumière venant des sources (7) est dirigée sur le fil (4) en mouvement en un seul endroit d'où elle est à la fois réfléchie et transmise et en ce que la lumière réfléchie est convertie en un signal électrique dans le premier capteur (14) et la lumière transmise est convertie en un signal électrique dans le second capteur (15) et où le premier capteur et le second capteur sont reliés à un additionneur (39).

5. Appareil selon la revendication 4, caractérisé en ce que des moyens (8, 12, 13, 9, 9', 10, 11) de transmission de lumière sont prévus entre la source de lumière (7) et le seul endroit fixe lelong du fil (4) en mouvement et entre celui-ci et les capteurs (14, 15).

6. Appareil selon la revendication 5, caractérisé en ce que les moyens de transmission de lumière (8, 12, 13) sont formés par des cavités réfléchissantes à l'intérieur et des prismes d'entrée (9, 9') ou de sortie (10, 11).

7. Appareil selon la revendication 6, caractérisé en ce qu'un premier transmetteur de lumière (8) qui dirige la lumière de la source (7) vers le fil (4) en passant par les prismes d'entrée (9, 9') comporte une fenêtre d'entrée (16), une face oblique (17) réfléchissante et un diviseur de rayons (18).

8. Appareil selon la revendication 6, caractérisé en ce que les moyens de transmission de lumière sont des photoconducteurs de n'importe quelle espèce.

9. Appareil selon la revendication 4, caractérisé en ce que la source de lumière (7) est connectée en série à un modulateur (25) pour son alimentation.

10. Appareil selon la revendication 9, caractérisé en ce que le modulateur (25) est relié à un régulateur (26) dont la constante du temps est ajustable et à un comparateur (27) qui compare une valeur donnée IS de l'intensité de la lumière à une valeur de sortie du démodulateur (34).

11. Appareil selon la revendication 4, caractérisé en ce que le premier capteur (14) et le second capteur (15) sont reliés à un deuxième ou quatrième filtre passe-haut (35 ou 36) par un premier ou troisième filtre passe-haut (31 ou 32) et un premier ou deuxième démodulateur (33 or 34) et en ce que ces éléments sont reliés par un amplificateur (37 ou 38) aux entrées d'un additionneur (39).

12. Appareil selon la revendication 11, caractérisé en ce que la sortie FF de l'additionneur (39) est reliée par un comparateur (40) qui compare la valeur d'un signal FS indiquant qu'aucune fibre étrangère n'est présente à la valeur du signal à la sortie de l'additionneur (39), à un second régulateur (41) qui règle l'amplificateur (37) variable.

13. Appareil selon la revendication 4, caractérisé en ce qu'un épurateur est logé en aval le long du fil (4) et est raccordé à des moyens électroniques (30) pour découper des parties du fil qui contiennent des fibres étrangères et pour raccorder des parties du fil.

14. Appareil selon la revendication 4, caractérisé en ce qu'une seule source de lumière (7) est prévue, de préférence ayant un spectre d'émission à large bande.

15. Appareil selon la revendication 14, caractérisé en ce que plusieurs capteurs (14) de lumière réfléchie sont prévus qui travaillent de préférence en parallèle.

16. Appareil selon la revendication 15, caractérisé en ce que les capteurs (14) ont des sensibilités dans différentes plages de longueur d'ondes.

17. Appareil selon la revendication 16, caractérisé en ce que plusieurs, de préférence deux sources (7) de lumière sont prévues.

18. Appareil selon la revendication 17, caractérisé en ce que chaque source (7) de lumière émet sa lumière dans sa propre plage de longueur d'ondes et de préférence en d'autres temps.

19. Appareil selon la revendication 18, caractérisé en ce que un seul capteur (14) de lumière à large bande est prévu.

20. Appareil selon la revendication 18, caractérisé en ce que un capteur (14) de lumière réfléchie est prévu correspondant à chaque source (7) de lumière qui émet dans sa propre plage de longueur d'ondes.

21. Appareil selon l'une des revendications 14 - 20, caractérisé en ce que un capteur (15) de lumière transmise est prévu qui est sensible dans une autre plage de longueur d'ondes que les capteurs (15) de lumière réfléchie.
